**Europäisches Patentamt**

**European Patent Office**

**Office européen des brevets**

(19)

(11) Veröffentlichungsnummer: **0 241 469 B1**

## EUROPÄISCHE PATENTSCHRIFT

(12)

(45) Veröffentlichungstag der Patentschrift: **23.10.91**

(51) Int. Cl.⁵: **A61K 9/50**

(21) Anmeldenummer: **86900659.3**

(22) Anmeldetag: **12.12.85**

(86) Internationale Anmeldenummer:
**PCT/EP85/00697**

(87) Internationale Veröffentlichungsnummer:
**WO 86/03675 (03.07.86 86/14)**

(54) **TEILCHEN AUS EINER HYDROPHOBEN ODER SCHWERLÖSLICHEN SUBSTANZ UND VERFAHREN ZU IHRER HYDROPHILISIERUNG.**

(30) Priorität: **14.12.84 CH 5941/84**

(43) Veröffentlichungstag der Anmeldung:
**21.10.87 Patentblatt 87/43**

(45) Bekanntmachung des Hinweises auf die
Patenterteilung:
**23.10.91 Patentblatt 91/43**

(84) Benannte Vertragsstaaten:
**AT BE CH DE FR GB IT LI LU NL SE**

(56) Entgegenhaltungen:
**AT-C- 368 880**
**US-A- 3 887 700**
**US-A- 4 344 934**

**See also references of WO8603675**

(73) Patentinhaber: **Gergely, Gerhard, Dr.**
**Gartengasse 8**
**A-1050 Wien(AT)**

(72) Erfinder: **GERGELY, Thomas**
**Gartengasse 8**
**A-1050 Wien(AT)**
Erfinder: **GERGELY, Irmgard**
**Gartengasse 8**
**A-1050 Wien(AT)**

(74) Vertreter: **Büchel, Kurt F., Dr.**
**Bergstrasse 297**
**FL-9495 Triesen(LI)**

## Beschreibung

Die Erfindung betrifft mikronisierte und hydrophilisierte Teilchen einer hydrophoben oder schwerlöslichen Substanz, die mit einem Tensid versetzt sind, sowie ein Verfahren zur Hydrophilisierung dieser Teilchen, insbesondere von pharmazeutischen Wirkstoffen, z.B. durch Benetzen der Teilchenoberfläche mit der Lösung eines Tensids und Abdampfen des Lösungsmittels von der Teilchenoberfläche.

Man hatte bisher zwar auf eine möglichst gute und homogene Mischung, bzw. Verteilung des Tensids auf dem Wirkstoff geachtet, dabei aber außer acht gelassen, daß die Verteilung nur im Makromaßstab als gut anzusehen war. Fur ein Auflösen oder Dispergieren solcher Teilchen in Wasser ist aber die Oberfläche jedes einzelnen oft nur 1 bis 5 μm grossen Teilchens, also der Mikromaßstab verantwortlich. Mit den bisher vorgeschlagenen Mischmethoden, z.B. nach der AT-A-368,880 oder der US-A-4,344.934, war es daher erforderlich, entweder verhältnismäßig große Tensidmengen (z.B. 0,15 bis 0,5 Teile pro 100 Teile Wirkstoff), und/oder sehr große Lösungsmittelmengen zu verwenden. Die erstere Massnahme bewirkt beim Eintrag der Teilchen in Wasser, insbesondere bei der Verwendung in Brausetabletten, ein unerwünscht starkes Schäumen, abgesehen davon, daß hohe Tensidmengen aus gesundheitlichen Gründen nicht erwünscht sind. Die zweite Maßnahme erfordert einen teuren Sprühtrocknungsvorgang, nach welchem ein Tensid zugesetzt wird, das sich wiederum nicht gleichmäßig verteilt.

In all diesen Fällen sind nämlich an einzelnen Stellen der Kornoberfläche der Substanzteilchen Tensidmoleküle agglomeriert, während andere Oberflächenstellen unbedeckt bleiben, da die auf der oft großen Oberfläche haftende Luft den Kontakt mit den Tensidmolekülen hindert. Beim Kontakt mit Wasser geht dann zunächst der an einzelnen Stellen der Kornoberfläche konzentrierte Tensidüberschuß in Lösung und bewirkt während des weiteren Vorgehens ein unerwünschtes Schäumen; die Teilchen mit der überwiegend hydrophoben und/oder schwerlöslichen Oberfläche, an der nur an wenigen Stellen Tensid haftet, werden aber nur schlecht dispergiert, weil das Tensid auf Wirkstoff bezogen zwar überschüssig, aber in der wässrigen Lösung viel zu verdünnt ist und daher nicht mehr zur Wirkung kommt.

Ähnliche Teilchen wurden auch in der US-A-3,887,700 beschrieben. Dort liegt allerdings nicht der schwer- oder unlösliche Wirkstoff (Aspirin®) allein, sondern dieser Wirkstoff in einer Mischung mit 33 - 100 % leicht löslichem Füllstoff, wie z.B. Mannitol oder Glycin vor. In dieser Mischung ist das Tensid aber durch einen Sprühtrocknungsvorgang mehr oder weniger gleichmässig verteilt und sitzt keineswegs an der Oberfläche der Wirkstoffteilchen. Das Lösungsverhalten ist daher ungünstig, wenn nicht sehr viel höhere Tensidmengen eingesetzt werden. Ausserdem kommt es durch die hohe Temperatur beim Sprühtrocknen meist zu einer unerwünschten, wenigstens teilweisen Verseifung des Aspirins R.

Die Erfindung hat sich daher die Aufgabe gestellt, hydrophobe oder schwerlösliche Teilchen zu hydrophilisieren, was durch die in den Ansprüchen 1 bis 5 angegebenen Maßnahmen erreicht wird, die besonders zweckmässig durch die in den Ansprüchen 6 bis 11 gegebenen Verfahrensschritte zu bewerkstelligen sind.

Grundsätzlich ist es zwar auch möglich, einen nahezu gleichmäßigen Überzug auf den Substanzteilchen zu erzielen, wenn Tensid in sehr großen Lösungsmittelmengen auf die Teilchenschüttung aufgebracht und diese unter ständigem Rühren getrocknet wird. Dazu sind aber viel Zeit und Energie erforderlich, und die Rückgewinnung großer Lösungsmittelmengen ist nicht angenehm.

Eine Reihe organischer Substanzen zeigt trotz annehmbarer Benetzbarkeit oder ausreichender Wasserlöslichkeit ein gewisses hydrophobes Verhalten Wasser gegenüber. Wirkstoffe, wie gewisse Antibiotika, die sich nicht in Wasser lösen, sondern suspendiert werden sollen, brauchen daher eine gewisse Zeit, bis sie an der Kristalloberfläche gewisse Wassermengen adhäsiv binden, so dass sie nach bestimmter Behandlungszeit in Wasser frei schweben. Sogar bei einigen wasserlöslichen Substanzen, wie etwa bei der relativ leicht wasserlöslichen Acetylsalicylsäure, bedarf es trotzdem einer gewissen Zeit, um sie - speziell in kaltem Wasser - in Lösung zu bringen. Ähnliches Verhalten zeigen Paracetamol und andere Wirkstoffe.

Um diese Erscheinungen zu beheben oder zu verbessern, hat man immer schon versucht, mit oberflächenaktiven bzw. waschaktiven Substanzen die Lösungs- bzw. Verteilungsfähigkeit solcher Wirkstoffe zu verbessern.

Da hiebei relativ grosse Mengen solcher Substanzen angewendet wurden, kam es speziell bei Verwendung der Wirkstoffe in Brausetabletten zu einem unliebsamen Schäumen und unansehnlichen Auflösungsbildern. Es wurde auch schon versucht, Substanzen waschaktiver Natur beim Umkristallisieren oder bei der Endreinigung von Wirkstoffen mit einzubauen. Diese Bemühungen blieben aber ohne Erfolg. Tenside bleiben nämlich beim Auskristallisieren der Wirkstoffe zunächst in der Mutterlauge und werden von der übrigen Verunreinigungen in grob verteilter Form ausgesalzt, ohne in dünner Schicht gleichmässig auf der Wirkstoffoberfläche zu haften. Die Filmbildung ist zugleich mit dem Umkristallisieren, dem Reinigen oder auch dem Waschen des Kristalles nicht erzielbar.

Es ist jedoch, insbesondere - aber nicht nur - durch Anwendung von Vakuum möglich, Wirkstoffsubstanzen zu erzielen, wie sie in den Ansprüchen 1 bis 5 beschrieben sind. Dazu gehört auch, daß man vorzugsweise bei jeder Substanz verschiedene Lösungsmittel, Gemische, und auch verschiedene Konzentrationen anbringt, damit eine optimale Filmbildung an der Oberfläche des Kristalls entsteht.

Die vorliegende Erfindung schlägt ein einfaches Verfahren vor, wobei nur Spuren von waschaktiven Substanzen angewendet werden müssen, um ausreichende Effekte zu erzielen. Die Verteilung von Substanzspuren auf einem feinteiligen Träger gelingt nämlich besonders gut im Vakuum durch das Entfernen der die Kristalle umgebenden Luftschichte unter speziellen Bedingungen des Aufbringens.

Bringt man nämlich eine gelöste Substanz, wie beispielsweise Dioctylsulfosuccinat in Methylenchlorid, auf beispielsweise Erythromycinsuccinat einer Grösse von 5-10 $\mu$m auf, und verdampft man das Methylenchlorid, dann wird es ohne geeignete Massnahmen in bekannter Weise zur Konzentrierung der sehr verdünnten Lösung kommen, so daß beim Trocknen Inseln gebildet werden, die am Ende dazu führen, daß ein geringer Teil der Kristalle relativ viel Tensidsubstanz bekommt, während der Rest unbedeckt bleibt.

Durch das erfindungsgemässe Verfahren gelingt es, die Tenside im ppm-Bereich auf Stoffe gleichmäßig aufzubringen und solcherart das Lösungs- oder Verteilungsverhalten von Substanzen zu verändern, ohne aber deren Zusammensetzung oder physiologisches Verhalten entscheidend zu verändern.

Gemäß einer Ausführungsform des erfindungsgemäßen Verfahrens kann zuerst die Gesamtmenge der Lösung durch das Bett der Substanz hindurchgesaugt und sodann ein Luftstrom durch das Bett hindurchgeleitet werden, bis das Lösungsmittel verdampft ist. Das Lösungsmittel kann auch ohne Luftstrom bei Unterdruck abgedampft werden.

Eine Variante des erfindungsgemäßen Verfahrens besteht darin, daß die Lösung in einen durch das Bett der zu hydrophilisierenden Substanz hindurchgeführten Luftstrom an der Eintrittsstelle in einen Vakuumkessel eingetropft wird; beim Durchtritt des aus Lösung und Luft bestehenden Gemisches durch das Bett wird durch die im Vakuum entstehende Verwirbelung und unter gleichzeitigem Rühren das Gemisch verteilt und das Lösungsmittel kontinuierlich verdampft. Man kann gegebenenfalls nach Verbrauch der Lösung noch weiter Luft durch das Bett hindurchführen, um allenfalls noch anhaftende Lösungsmittelmengen von der hydrophilisierten Substanz abzudampfen.

Sollten es diese Verfahrensbedingungen erfordern, kann die als Verdampfungsmittel oder als Trägergas für die Lösung verwendete Luft vorgewärmt werden. An Stelle von Luft kann im Falle von sauerstoffempfindlichen Substanzen ein gegenüber diesen inertes Gas, wie Stickstoff, eingesetzt werden.

Bei Verwendung der Luft als Trägergas für die Lösung wird vorzugsweise die Luft, welche die Lösung in einem Anteil von 5 bis 10 Masse-% der zu hydrophilisierenden Substanz enthält, in einer Menge von 100 bis 500 l/min. pro 100 kg der zu hydrophilisierenden Substanz durch das Bett hindurchgeführt.

Als Antischaummittel wird vorzugsweise eine Flüssigkeit mit einem Dampfdruck von unter 2 mbar bei 25 Grad C eingesetzt, wie insbesondere ein höherwertiger Alkohol, ein Aldehyd oder ein Keton.

Die zusammen mit dem Tensid auf die zu hydrophilisierende Substanz aufgebrachte geringe Menge an Klebemittel, wie z.B. Polyvinylpyrrolidon oder Carboxymethylcellulose, dient dazu, das Tensid auf der Oberfläche der Substanz zu fixieren.

Durch Verwendung der äusserst geringen Mengen an Tensid wird ein Schäumen und die daraus resultierende Blasenbildung, insbesondere im Falle der Verwendung der hydrophilisierten Substanzen in Brausetabletten, weitestgehend reduziert. Um die erwähnte Blasenbildung sicher und vollständig zu unterdrücken, hat es sich als vorteilhaft erwiesen, zugleich mit dem Tensid und dem Klebemittel als Antischaummittel eine Flüssigkeit mit einem niedrigen Dampfdruck von unter 2 mbar bei 25 $^\circ$C auf die zu hydrophilisierende Substanz aufzubringen. Beispiele dafür besonders geeigneter Verbindungen sind höhermolekulare Alkohole, Aldehyde und Ketone, z.B. Benzylalkohol.

Durch das erfindungsgemäße Hydrophilisierungsverfahren kann also die Suspensionsfähigkeit wasserunlöslicher Substanzen, wie beispielsweise Carbocistein, Amoxycillin oder Erythromycinsuccinat verbessert werden. Auch kann die Lösungsgeschwindigkeit schwerlöslicher Substanzen in Wasser erhöht werden, wenn solche Substanzen mit geringsten Mengen Tensid und Klebemittel behandelt werden, wie es beispielsweise bei Paracetamol, Acetylsalicylsäure oder Ampicillin, der Fall ist. Diese Substanzen zeigen nach der Behandlung eine sofortige Immersion in Wasser, d.h., sie bleiben nicht oben schwimmen, sondern sie gehen unter und suspendieren oder lösen sich unter leichtem Rühren sofort.

Es kann nach dem erfindungsgemäßen Verfahren grundsätzlich jede Substanz, deren Lösung oder Suspendierung in Wasser beabsichtigt ist, hydrophilisiert werden. In besonderer Weise bietet sich jedoch das erfindungsgemäße Verfahren für in Wasser zu suspendierende oder zu lösende pharmazeutische Wirkstoffe an. Diese hydrophilisierten Wirkstoffe können beispielsweise auch in Pulvergemischen für die Herstellung von Instantgetränken oder in Brausetabletten enthalten sein.

Im Falle der Instantprodukte kann man die hydrophilisierten Substanzen, wenn sie zusätzlich ein Bindemittel, wie beispielsweise Polyvinylpyrrolidon, enthalten, an Zuckerkristalle durch einfaches Mischen anlegen lassen. Durch dieses Adhäsionsvermögen gelingt es beispielsweise mittels eines einfachen Mischvorganges verhältnismässig leicht, Überzüge solcher Substanzen auf Kohlehydratkristallen herzustellen. Die hydrophilisierten Substanzen bilden auf diese Weise vorbehandelte Stoffe, welche dann bei trockener Mischung mit Zuckerkristallen ein Adhäsionsvermögen zeigen und vom Zuckerkristall herunter in Suspension gehen.

Dies gilt nicht nur für die bereits erwähnten Antibiotika, sondern z.B. auch für Paracetamol. Unbehandeltes Paracetamol in einer Brausetablette neigt sehr zum Schäumen, die Auflösungsgeschwindigkeit ist wesentlich verlangsamt, wodurch Partikel an die Oberfläche gelangen, die sich nicht lösen, einen Film an der Getränkeoberfläche bilden und zu einer hässlichen Schaumbildung neigen, während hydrophilisiertes Paracetamol sich gleichzeitig mit der Brausemischung klar löst. Dasselbe gilt für nicht oberflächenbehandelte Acetylsalicylsäure, die in Brausemischungen verarbeitet wird, so dass beim Auflösen der Brause dann die Kristalle an der Oberfläche schwimmen, sich am Glasrand anlegen, während bei dem behandelten Wirkstoff die Kristalle in das Wasser einsinken und sich in der Lösung anschliessend klar auflösen.

Als Tenside können im erfindungsgemäßen Verfahren anionische, kationische oder nichtionische Tenside eingesetzt werden. Beispiele geeigneter Tenside sind Dioctylsulfo-succinat und Polysorbat.

Die Erfindung wird durch die folgenden Beispiele näher erläutert. In den Beispielen wurde ein mit Rührer, Heizmantel und Austragsieb ausgerüsteter, schwenkbarer Vakuumkessel verwendet. Ein für die erfindungsgemäßen Zwecke besonders geeigneter Vakuumkessel ist in den AT-B-375 279 und 376 147 beschrieben. In den Beispielen sind alle Teil- und Prozentangaben, soweit nicht anders angegeben, auf die Masse bezogen.

Beispiel 1:

100 Teile mikronisiertes Erythromycinsuccinat, Kristallgrösse 5-10 μm, werden mit 0,1 Teilen Dioctylsulfosuccinat und 3 Teilen Polyvinylpyrrolidon, gelöst in 8 Teilen Alkohol, behandelt.

Das Erythromycinsuccinat wird bei einer Manteltemperatur von 50 °C auf 40 °C erhitzt. Sodann wird auf ca. 50 mbar evakuiert und unter dreidimensionalem Rühren (Swinging-Rühren) mit höchster Geschwindigkeit die Lösung langsam eingesaugt. Der Einsaugvorgang soll etwa 3-5 min dauern.

Sodann wird unter Beibehalten des intensiven Rührens bis auf einen Endwert von 20 mbar evakuiert. Das Erythromycinsuccinat wird dann z.B. über ein rotierendes Sieb ausgetragen.

Beispiel 2:

100 Teile mikronisiertes Ampicillin-anhydrid werden mit 0,01 Teilen Dioctylsulfosuccinat und 1 Teil Polyvinylpyrrolidon, gelöst in 4 Teilen Alkohol, behandelt. Die weitere Behandlung erfolgt wie unter Beispiel 1.

Beispiel 3:

100 Teile Amoxycillin-trihydrat werden mit 0,1 Teilen "Tween"® (Polysorbat 80), 2 Teilen Polyvinylpyrrolidon und 2 Teilen Äthylcellulose kurzer Kettenlänge, in 4 Teilen Alkohol und 2 Teilen Methylenchlorid gelöst, behandelt.

Da Amoxycillin-trihydrat empfindlich auf Trocknungsverluste ist, wird die Behandlung im Luftstrom kontinuierlich durchgeführt. Die Manteltemperatur beträgt nur 40 °C, und es wird bei einem Unterdruck von 800 mbar unter intensivem Rühren (dreidimensional) ein Luftstrom von 100 Teilen Luft/min durchströmen gelassen (bei 100 kg Masse sind es also 100 l Luft/min).

Unmittelbar vor der Einströmdüse wird durch ein Vorratsgefäss die obenerwähnte Lösung in den Luftstrom eingebracht, so dass die Gesamtlösung in etwa 5 min über den Luftstrom in der Masse verteilt wird. Etwa 70-80% des Lösungsmittels verdampfen während dieser Zeit. Man muß daher weiteren Luftstrom etwa 10-15 min lang nachströmen lassen, um den Rest des Lösungsmittels zu verdampfen. Reste von adsorbiertem Lösungsmittel werden durch völliges Evakuieren der Masse ohne Luftstrom auf 10-20 mbar entfernt.

Beispiel 4:

100 Teile Carbocistein werden mit 0,1 Teilen Dioctylsulfosuccinat und 2 Teilen Polyvinylpyrrolidon in einer Lösung von 5 Teilen Methylenchlorid behandelt. Da Methylenclorid besonders schnell verdampft, empfiehlt sich die Behandlung in strömender Luft.

Carbocistein wird daher bei einer Manteltemperatur von 50 Grad C auf 40 Grad C vorgeheizt. Man evakuiert den Kessel und saugt bei 800 mbar einen Luftstrom von 100 Teilen Luft/Minute bei starkem und dreidimensionalem Rühren durch die Masse.

In diesen Luftstrom wird mit einer Geschwindigkeit von 1 Teil Lösung/Minute die Lösung eingebracht. Sie kondensiert und verdampft kontinuierlich auf dem Carbocistein.

In diesem Fall kann man die Luft auf 40 Grad C vorheizen, so dass durch die Verdampfungswärme des Methylenchlorids kein Temperaturverlust entsteht. Nach etwa 5 Minuten ist die gesamte Lösung eingebracht; es wird weitere 3 Minuten heftig unter weiterem Luftdurchleiten gerührt und sodann zur Beseitigung der Restmengen Methylenchlorid auf 10 mbar evakuiert.

Beispiel 5:

100 Teile Paracetamol (Korngrösse 100 μm) werden mit 0,01 Teilen Dioctylsulfosuccinat, 1 Teil Polyvinylpyrrolidon und 0,5 Teilen Benzylalkohol behandelt, die in 12 Teilen Methylenchlorid gelöst sind.

Man heizt das Paracetamol auf 50 Grad C (bei einer Manteltemperatur von 60 Grad C) vor, evakuiert auf 20 mbar und bringt die Lösung durch Einströmen in die Masse bei etwa 50 mbar (bei abgesperrtem Ventil zur Vakuumpumpe) ein. Durch das verdampfende Lösungsmittel stellt sich ein allgemeiner Konstantdruck von etwa 200 mbar ein, und man rührt etwa 5 Minuten dreidimensional zur völligen Verteilung des Lösungsmittels.

Sodann evakuiert man unter heftigem Rühren bis auf 200 mbar, wobei ein Teil des Benzylalkohols verdampft, aber etwa 70% der Benzylalkoholmenge in dem Ueberzug am Paracetamol verbleiben.

Man evakuiert am Ende wiederum auf 10-20 mbar. Die durch das Sieb auszutragende Masse darf nicht agglomeriert sein.

Beispiel 6:

100 Teile Acetylsalicylsäure werden mit einer Lösung von 0,2 Teilen Polyvinylpyrrolidon und 0,03 Teilen Dioctylsulfosuccinat in 6 Teilen Äthylalkohol behandelt.

Man kann sowohl das Verfahren unter Beispiel 1 als auch das unter Beispiel 3 anwenden. Bei größeren Mengen empfiehlt sich jedoch die nichtkontinuierliche Vorgangsweise, da diese Überzüge auf Acetylsalicylsäure schwerer herstellbar sind.

Demnach werden 100 Teile Acetylsalicylsäure bei einer Manteltemperatur von 55 °C auf 45 °C vorgeheizt und dreidimensional heftig gerührt. Man evakuiert auf 20 mbar und bringt sodann die Lösung so ein, dass die Gesamtlösung etwa 5 min zum Einströmen braucht. Man mischt weitere 5 min wie vorher und verdampft hernach durch Öffnung des Ventils zur Vakuumpumpe. Hier ist es vorteilhaft, bei dem Verdampfen des Lösungsmittels 100 Teile Luft/min durchströmen zu lassen, bei einem Konstantvakuum von 600-800 mbar. Durch dieses Luftdurchströmen wird das Lösungsmittel gleichmäßig entfernt; die Turbulenz des Luftstrahls bewirkt aber eine Aufrechterhaltung des Films von Dioctysulfosuccinat und Polyvinylpyrrolidon, was gerade bei Acetylsalicylsäure schwierig ist und besondere Vorsichtsmaßregeln verlangt.

Nach etwa 10 min Durchströmen der Luft bei Konstantvakuum wird das Einsaugventil zur Luft verschlossen und bis zu einem Endwert von 10 mbar evakuiert.

Beispiel 7:

Man kann bei der Herstellung von Brausetabletten so vorgehen, dass eine Brausetablettenmischung mit der unter vorgenanntem Beispiel oberflächenbehandelten Acetylsalicylsäure gemischt und verpreßt wird. Solche Tabletten zeigen beim Auflösen nicht den üblichen unangenehmen Rand unaufgelöster Kristalle am Glas, sondern es ergibt sich ein völlig gleichmäßiges Auflösungsbild; die Acetylsalicylsäure löst sich gleichzeitig mit der Brausemischung im Wasser klar auf.

Beispiel 8:

In anderen Fällen kann man das erfindungsgemäße Verfahren verwenden, um gleichzeitig mit der Behandlung der Wirkstoffoberfläche eine Granulierung durch Herstellung eines Überzuges auf einem Trägerstoff durchzuführen:

100 Teile Zucker werden mit 10 Teilen Carbocistein versetzt; die Mischung wird im Vakuumkessel auf 50 °C erhitzt. Der Zukker hat vorzugsweise eine Kristallgröße von 0,3-0,2 mm, während das Carbocistein 5-10 μm Kristallgröße haben soll.

Sodann wird auf 20 mbar evakuiert und eine Lösung von 0,1 Teil Dioctylsulfosuccinat mit 2 Teilen Polyvinylpyrrolidon in 6 Teilen Alkohol im Verlauf von 2 min. gleichmäßig auf die gesamte Masse eingetragen.

Nach weiteren 3 min dreidimensionaler Verteilung wird das Vakuumventil geöffnet und der Alkohol verdampft.

Man kann die weitere Anklammerung des Carbocisteins an den Zucker dadurch erreichen, dass man unter denselben Bedingungen anschliessend noch 2% einer wässrigen Zuckerlösung, bestehend aus 1 Teil Wasser und 1 Teil Zucker, in den Vakuumkessel einbringt. Man evakuiert auf 50 mbar und lässt diese konzentrierte Zuckerlösung wiederum während einer Zeit von 2-3 min einströmen.

Nach weiteren 3 min. dreidimensionalen Rührens wird voll evakuiert, bis ein Endvakuum von 20 mbar erreicht wird.

Diese Masse kann mit Farbstoffen, Geschmackskorrigentien und Süßstoffen versehen werden und gibt ein Granulat, welches nach Einbringen in Wasser Carbocistein freischwebend in der Suspension hält.

Beispiel 9:

1000 mg hydrophober mikronisierter Wirkstoff, z.B. eines der in den vorangehenden Beispielen genannten Antibiotika, werden in einer Schale mit 0,1 mg Dioctylsulfosuccinat und 30 mg Polyvinylpyrrolidon in 80 mg Alkohol kräftig verrieben. 100 mg des anschliessend getrockneten Pulvers werden in 100 ml Wasser gegeben und man rührt kurz um: Es bleiben noch nach 3 min schlecht benetzte Teilchen und agglomerierte Klümpchen, teilweise auf der Wasseroberfläche, teilweise abgesunken, übrig.

100 mg desselben Wirkstoffes werden in 100 ml Wasser gegeben, dem nur 0,01 mg (!) Dictylsulfosuccinat zugesetzt wurden. Der Wirkstoff bleibt größtenteils unbenetzt und schlecht verteilt; der Unterschied zu reinem Wasser ist vernachlässigbar.

100 mg des nach Beispiel 1 behandelten Wirkstoffes werden auf die Oberfläche von 100 ml Wasser gegeben. Sie sinken - in einzelne Teilchen getrennt - innerhalb von 30 sec zu Boden und ergeben nach kurzem Umrühren eine angenehm zu trinkende Suspension.

**Patentansprüche**

1. Mikronisierte und hydrophilisierte Pulverteilchen einer hydrophoben oder schwerlöslichen Substanz, die mit einem Tensid versetzt sind, dadurch gekennzeichnet, daß die Oberfläche der Teilchen von einer zusammenhängenden Schicht von maximal 0,1 Gewichtsteilen Tensid in maximal 10 Gewichtsteilen eines Bindemittels (auf 100 Gewichtsteile der Substanz bezogen) bedeckt ist.

2. Teilchen nach Anspruch 1, dadurch gekennzeichnet, daß sie von maximal 0,05 Gewichtsteilen Tensid in maximal 6 Gewichtsteilen Bindemittel bedeckt sind.

3. Teilchen nach Anspruch 1 oder 2, zur Herstellung von Instantgranulat oder -tabletten, dadurch gekennzeichnet, daß sie aus einem schwerlöslichen oder unlöslichen pharmazeutischen Wirkstoff, insbesondere aus Erythromycinsuccinat, Amoxicillin, Penicillin oder Carbocistein bestehen.

4. Teilchen nach Anspruch 1 oder 2, zur Herstellung von Brausegranulat oder -tabletten, dadurch gekennzeichnet, daß sie aus einem hydrophoben, mittel- bis leichtlöslichen pharmazeutischen Wirkstoff, insbesondere aus Acetylsalicylsäure oder Paracetamol bestehen.

5. Teilchen nach einem der vorangehenden Ansprüche, dadurch gekennzeichnet, daß sie mittels eines Bindemittels an Kohlehydratkristalle angelagert sind.

6. Verfahren zur Herstellung von Teilchen nach einem der Ansprüche 1 bis 5, dadurch gekennzeichnet, daß man durch eine Schüttung der Teilchen bei einem Druck von unter 800, vorzugsweise unter 100 mbar eine Lösung saugt, die - bezogen auf die Masse der zu hydrophilisierenden Teilchen - wenigstens ein Tensid in einer Menge von 0,01 bis 0,1 Masse-%, und ein Bindemittel in einer Menge entsprechend dem 6,6- bis 100-fachen der Masse des eingesetzten Tensids enthält.

7. Verfahren nach Anspruch 6, dadurch gekennzeichnet, daß die Gesamtmenge der Lösung durch die Schüttung der Teilchen hindurchgesaugt und sodann ein Luftstrom durch das Bett hindurchgeleitet wird.

8. Verfahren nach Anspruch 6, dadurch gekennzeichnet, daß die Lösung in einen durch die Schüttung der Teilchen hindurchgeführten Luftstrom an der Eintrittsstelle in den Vakuumkessel eingetropft und bei Durchtritt des aus der Lösung und Luft bestehenden Gemisches durch die Schüttung mittels der im Vakuum entstehenden Verwirbelung das Gemisch gleichzeitig unter Rühren verteilt und das Lösungsmittel kontinuierlich abgedampft wird.

9. Verfahren nach Anspruch 8, dadurch gekennzeichnet, daß die Luft, welche die Lösung in einem Anteil von 5 bis 10% der Teilchenmasse enthält, in einer Menge von 100 bis 500 1/min. pro 100 kg der Teilchen durch die Schüttung hindurchgeführt wird.

10. Verfahren nach einem der Ansprüche 6 bis 9, dadurch gekennzeichnet, daß die Teilchen zusätzlich mit einem Antischaummittel in einer Menge von 0,01 bis 1 Masse%, vorzugsweise mit einem höhermolekularen Alkohol, einem Aldehyd oder einem Keton mit einem Dampfdruck bei 25 Grad C unter 2 mbar versetzt werden.

11. Verfahren nach einem der Ansprüche 6 bis 10, dadurch gekennzeichnet, daß bei Aufrechterhaltung des Unterdruckes, gleichzeitig mit und/oder nach dem Einsaugen der Lösung bis zur vollständigen Verdampfung des Lösungsmittels ein Luftstrom hindurchgeleitet oder - gesaugt, und/oder der Druck auf unter 50, vorzugsweise unter 20 mbar abgesenkt wird.

## Claims

1. Micronized and hydrophilized particles of powder of a hydrophobic or poorly soluble substance, which are mixed with a surfactant, characterized in that the surface of the particles is covered by a continuous layer of a maximum of 0,1 parts by weight of surfactant in a maximum of 10 parts by weight of a binder (referred to 100 parts by weight of the substance).

2. Particles as in Claim 1, characterized in that they are covered by a maximum of 0,05 parts by weight of surfactant in a maximum of 6 parts by weight of binder.

3. Particles as in Claim 1 or 2, for the production of instant granulate or instant tablets, characterized in that they consist of a poorly soluble or insoluble pharmaceutical active substance, in particular of erythromycinsuccinate, amoxicillin, penicillin or carbocistein.

4. Particles as in Claim 1 or 2, for the production of spray granulate or spray tablets, characterized in that they consist of a hydrophobic medium-to-readily soluble pharmaceutical active substance, in particular of acetylsalicylic acid or paracetamol.

5. Particles as in one of the preceding Claims, characterized in that they are deposited by means of a binder onto carbohydrate crystals.

6. A method of production of particles as in one of the Claims 1 to 5, characterized in that at a pressure of below 800, preferably below 100 mbar one sucks through a pile of the particles a solution which - referred to the weight of particles to be hydrophilized - contains at least one surfactant in an amount of from 0.01 to 0.1 % by weight and a binder in an amount corresponding with 6.6 to 100 times the weight of surfactant used.

7. A method as in Claim 6, characterized in that the whole of the solution is sucked through the pile of particles and then a flow of air is passed through the bed.

8. A method as in Claim 6, characterized in that the solution is dripped at the point of entry into the vacuum vessel into a flow of air being led through the pile of particles and upon the mixture consisting of the solution and air passing through the pile the mixture is simultaneously distributed by stirring by means of the turbulence arising in the vacuum and the solvent is continuously evaporated.

9. A method as in Claim 8, characterized in that the air which contains the solution in a proportion of from 5 to 10 % of the weight of particles, is led through the pile in an amount of from 100 to 500 1/min. per 100 kg of particles.

10. A method as in one of the Claims 6 to 9, characterized in that the particles are in addition mixed with an antifoaming agent in an amount of from 0.01 to 1 % by weight, preferably with a higher molecular alcohol, an aldehyde or a ketone having a vapour pressure at 25 degrees C below 2 mbar.

11. A method as in one of the Claims 6 to 10, characterized in that with maintenance of the reduced pressure, simultaneously with and/or after the sucking in of the solution until complete evaporation of the solvent a flow of air is passed or sucked through and/or the pressure is lowered to below 50, preferably to below 20 mbar.

## Revendications

1. Particules de poudre micronisées et hydrophilisées d'une substance hydrophobe ou peu soluble, qui sont additionnées d'un agent tensioactif, caractérisées en ce que la surface des particules est recouverte d'une couche continue d'au maximum 0,1 partie en poids d'agent tensioactif dans au maximum 10 parties en poids d'un liant (pour 100 parties en poids de la substance).

2. Particules suivant la revendication 1, caractérisées en ce qu'elles sont recouvertes de 0,05 partie en poids au maximum d'un agent tensioactif dans 6 parties en poids au maximum d'un liant.

3. Particules suivant les revendications 1 ou 2, pour la préparation de granulés ou de comprimés instantanés, caractérisées en ce qu'elles se composent d'une substance active pharmaceutique peu soluble ou insoluble, en particulier de succinate d'érythromycine, d'amoxicilline, de pénicilline ou de carbocistéine.

4. Particules suivant les revendications 1 ou 2, pour la préparation d'un granulé ou de comprimés effervescents, caractérisées en ce qu'elles se composent d'une substance active pharmaceutique hydrophobe, moyennement soluble à très soluble, en particulier d'acide acétylsalicylique ou de paracétamole.

5. Particules suivant l'une des revendications précédentes, caractérisées en ce qu'elles sont déposées au moyen d un liant sur des cristaux d'hydrate de carbone.

6. Procédé de préparation de particules suivant l'une des revendications 1 à 5, caractérisé en ce qu'on aspire à travers un tas des particules, sous une pression inférieure à 800, de préférence inférieure à 100 mbar, une solution qui contient, par rapport à la masse des particules à hydrophiliser, au moins un agent tensioactif dans une quantité de 0,01 à 0,1% de la masse, et un liant dans une quantité correspondant à 6,6 à 100 fois la masse de l'agent tensioactif utilisé.

7. Procédé suivant la revendication 6, caractérisé en ce que la totalité de la solution est aspirée à travers le tas des particules, puis en ce qu'on fait passer un courant d'air à travers le lit.

8. Procédé suivant la revendication 6, caractérisé en ce que la solution est introduite goutte à goutte dans un courant d'air envoyé à travers le tas de particules à l'emplacement d'entrée dans le récipient à vide, et en ce que par passage du mélange constitué de la solution et d'air à travers le tas, au moyen du tourbillon se formant dans le vide, le mélange est simultanément réparti avec agitation et le solvant est évaporé en continu.

9. Procédé suivant la revendication 8, caractérisé en ce que l'air que contient la solution dans une proportion de 5 à 10 % de la masse des particules, est envoyé à travers le tas dans une quantité de 100 à 500 l/min. pour 100 kg des particules.

10. Procédé suivant l'une des revendications 6 à 9, caractérisé en ce que les particules sont en outre additionnées d'un anti-mousse dans la quantité de 0,01 à 1 % de la masse, de préférence d'un alcool de masse moléculaire élevée, d'un aldéhyde ou d'une cétone ayant une tension de vapeur à 25 degrés C inférieure à 2 mbar.

11. Procédé selon l'une des revendications 6 à 10, caractérisé en ce que, avec maintien de la dépression, un courant d'air est envoyé ou aspiré, en même temps que l'aspiration de la solution ou après celle-ci, jusqu'à évaporation complète du solvant, et/ou la pression est abaissée au-dessous de 50, de préférence au-dessous de 20 mbar.